(19) Europäisches Patentamt
European Patent Office
Office européen des brevets

(11) Publication number: **0 677 511 A2**

(12) **EUROPEAN PATENT APPLICATION**

(21) Application number: **95201522.0**

(22) Date of filing: **29.05.84**

(51) Int. Cl.[6]: **C07C 315/04**, C07C 319/20, C07D 263/26, C07C 317/32, C07C 323/41

This application was filed on 09 - 06 - 1995 as a divisional application to the application mentioned under INID code 60.

(30) Priority: **02.06.83 IT 214783**
**05.08.83 IT 2244983**
**03.02.84 IT 1943584**

(43) Date of publication of application:
**18.10.95 Bulletin 95/42**

(60) Publication number of the earlier application in accordance with Art.76 EPC: **0 130 633**

(84) Designated Contracting States:
**AT BE CH DE FR GB IT LI LU NL SE**

(71) Applicant: **ZAMBON S.p.A.**
**Via della Chimica, 9**
**I-36100 Vicenza (IT)**

(72) Inventor: **Jommi, Giancarlo**
**Via Gozzano, 4**
**I-20131 Milano (IT)**
Inventor: **Chiarino, Dario**
**Via Rivolta, 2**
**I-20052 Monza (Milano) (IT)**
Inventor: **Pagliarin, Roberto**
**Via Zara, 29**
**I-20010 San Giorgio su Legnano (Milano) (IT)**

(74) Representative: **Marchi, Massimo et al**
**c/o Marchi & Mittler s.r.l.**
**Viale Lombardia 20**
**I-20131 Milano (IT)**

(54) **Intermediates for the preparation of 1-(phenyl)-1-hydroxy-2-amino-3-fluoro propane derivatives.**

(57) A process for preparing a D-threo compound of the formula

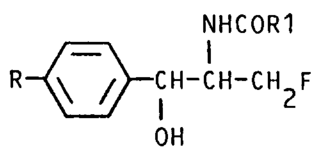

(II)

wherein R, and R1 have the meanings shown in the description, via protection of both the secondary hydroxy and the amino group of a corresponding D-threo 1-(phenyl)-2-amino-1,3-propanediol, fluorination of the protected compound and treatment of the thus obtained compound to afford a D-threo compound of the formula (II).

EP 0 677 511 A2

This invention relates to new intermediates useful for preparing 1-(phenyl)-1-hydroxy-2-amino-3-fluoro-propane derivatives.

More particularly this invention relates to new Compounds of Formula

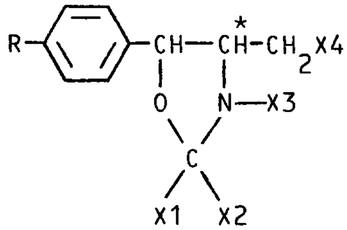

(I)

where

R is a methylthio, methylsulfoxy, methylsulfonyl or a nitro group; and

X1 is hydrogen, 1-6 C alkyl, 1-6 C haloalkyl, 3-6 C cycloalkyl, phenyl or phenylalkyl(1-6C), where the phenyl ring may be substituted by one or two halogen, 1-3 C alkyl, 1-3 C alkoxy or nitro; or
X1 together with X2 is an oxygen atom or an alkylene having from two to five Carbon atoms; or
X1 together with X2 and R4 is a chain of formula

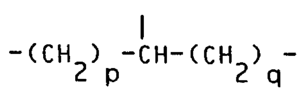

where p is 3 or 4 and q is 1 or 2; and

X2 is hydrogen, 1-6 C alkyl, 1-6 C haloalkyl, 3-6 C cycloalkyl or phenyl which may be substituted by one or two halogen, 1-3 C alkyl, 1-3 C alkoxy or nitro; or
X2 together with X1 has the above mentioned meanings; or
X2 together with X3 is a covalent bond; or
X2 together with R4 is
$-(CH_2)_m-CH=CH-$ , $-(CH_2)_m-CH_2-CH_2-$

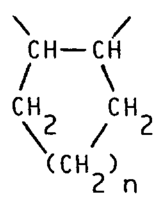

or

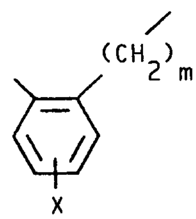

where n is 1 or 2; m is 0 or 1; X is hydrogen, halogen, 1-3 C alkyl, 1-3 C alkoxy or nitro; or X2 together with X1 and R4 has the above mentioned meanings; and

X3 is hydrogen or -CO-R4 where R4 is hydrogen, 1-6 alkyl, 1-6 C haloalkyl, 3-6 C cycloalkyl, phenyl or phenylalkyl(1-6C) where the phenyl ring may be substituted by one or two halogen, 1-3 C alkyl, 1-3 C alkoxy or nitro; or
R4 together with X2 has the above mentioned meanings; or
R4 together with X2 and X1 has the above mentioned meanings; or
X3 together with X2 has the above mentioned meanings; and

X4 is -OH, OCOAlkyl(1-4C), fluorine, -O-trialkyl(1-6C) silyl, -O-tetrahydropyranyl, -O-tetrahydrofuranyl or $-O-SO_2R6$ where R6 is methyl, trifluoromethyl, phenyl or p-methyl-phenyl provided, however, that X4 is not OH when R is a nitro group.

which are useful as intermediates for preparing Compounds of Formula

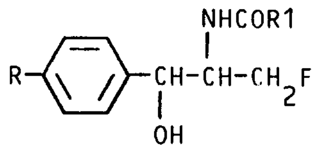

(II)

wherein R is $-NO_2$, $CH_3S-$, $CH_3SO-$, and $CH_3SO_2$; and

R1 is mono-, di-, and tri-halomethyl.

The Compounds of Formula II contain two asymmetric carbon atoms and can exist as stereoisomers. Unless otherwise specified herein or in the claims, it is intended to include all four stereoisomers, whether separated or mixtures thereof. The D-(threo)-forms are preferred because of their broader antibacterial activity.

U.S. Patent 4,235,892 discloses the Compounds of Formula II and a process for their preparation. This process essentially consists in N-protecting a 1-(phenyl)-2-amino-1,3-propanediol, (the phenyl moiety of which is variously substituted) by an imido derivative of a bicarboxylic acid, in treating the thus obtained compound with dialkylaminosulfotrifluoride (DAST), in removing the N-protecting group and then in acylating the thus obtained 1-(phenyl)-1-hydroxy-2-amino-3-fluoro-propane with the desired haloacetic acid or with a suitable reactive derivative thereof.

Although apparently easy, this process suffers many disadvantages and affords low yields.

One of the main disadvantages is that the fluorination of the primary hydroxy group is not selective and leads to the formation of many byproducts, among which may be mentioned, for instance, the products deriving from the substitution at the secondary hydroxy group. The desired compound may thus be obtained at a sufficient purity degree only by a particularly complex column chromatography process. Another disadvantage is that DAST is the only agent which allows to perform the fluorination step on the peculiar intermediate products which are prepared according to the process of US Patent 4,235,892 and DAST is very expensive and dangerous, especially when it is intended for large scale production.

It has been now found that the above mentioned disadvantages can be overcome by using new Compounds of Formula I wherein either the amino group or the secondary hydroxy group of the 1-(phenyl)-2-amino-1,3-propanediol derivative are protected.

Preferably also the primary hydroxy group is substituted by a leaving group which can be replaced by a fluorine atom.

The Compounds of Formula I have the advantage to avoid the formation of many by-products and this not only in that the protection hinders the fluorination of the secondary hydroxy group but also in that it insure the stability of the configuration of the asymmetric carbon atoms.

Furthermore the Compounds of Formula I are quite soluble in the most part of the aprotic organic solvents, thus allowing to carry out the fluorination step also in homogeneous phase and under anhydrous and mild conditions.

Another advantage is that the Compounds of Formula I are prepared very easily by making use of cheap reactants and that the protective groups can be removed very easily as well after the completion of the fluorination step.

Still another advantage of the Compounds of Formula I is that the protection of the secondary hydroxy group allows to substitute the primary hydroxy group with a suitable leaving group so that the fluorination step can be most conveniently carried out with fluorination agents which are cheaper and less dangerous than DAST.

The protection of the amino and of the secondary hydroxy group thus performed is substantially inert to those treatments that the molecule undergoes, more particularly with nucleophiles and bases, up to the fluorination step and can then be easily removed under mild conditions.

When X1, X2, and R4, equal or different among them, are hydrogen, 1-6 C alkyl, 1-6 C haloalkyl, 3-6 C cycloalkyl, phenyl or substituted phenyl, examples of suitable reactants are: aldehydes such as formaldehyde, acetaldehyde, valeraldehyde, caproaldehyde, benzaldehyde, anisaldehyde, 4-chlorobenzaldehyde, 4-ethoxy-3-methoxy-benzaldehyde, 2,6-di nitrobenzaldehyde or ketones such as acetone, diethylketone or hexylmethylketone for protecting the secondary hydroxy group and one hydrogen of the amino group, and acids as acetic, dichloroacetic, trifluoroacetic, pivaloyl, benzoic, 2,4-dibromobenzoic, veratric, 2,5-dimethylbenzoic or 4-nitrobenzoic acid, for protecting the second hydrogen of the amino group.

When X1 and X2, together, are an alkylene radical having from 2 to 5 carbon atoms, examples of suitable reactants are the cycloalkanones such as cyclopropanone, cyclopentanone or cyclohexanone.

When X1 together with X2 and R4 is a chain of formula $-(CH_2)_p$ $-CH-(CH_2)_q-$ where p and q have the above mentioned meanings, examples of suitable reactants are the ketoacids such as (2-oxocyclopentyl) acetic acid, (2-oxocyclohexyl)-acetic acid, 3-(2-oxocyclopentyl) propionic acid and 3-(2-oxocyclo hexyl)-propionic acid.

When X2 together with R4 forms a mono- or a poly-cylic system, examples of suitable reactants are the aldehydo-acids or the keto-acids such as phthalaldehydic acid, succinic semialdehyde, levulinic acid, 4-phenyl-4-oxo-butyric acid; hexahydrophthalaldehydic acid; (2-acetyl)-cyclohexylcarboxylic acid and (2-acetyl)-cyclopentyl-carboxylic acid.

When X1 together with X2 is an oxygen atom, examples of suitable reactants are the halocarbonates of formula $XCOOR_2$ where X is a halogen atom and R2 is an alkyl, aralkyl or an aryl radical; preferably R2 is an 1-4 C alkyl radical.

Preferred Compounds according to this invention are those wherein X2 and X3 together are a covalent bond and X1 is hydrogen, 1-6 C alkyl, 1-6 C haloalkyl, phenylalkyl(1-6C), phenyl where the phenyl ring may be substituted by one or two halogen, 1-3 C alkyl, 1-3 C alkoxy or nitro. Examples of suitable reactants for preparing such Compounds are the amidines of formula $X1-C(=NH)-NH_2$ or their salts, the halohydrates of iminoethers of formula $X1-C-(OR')=NH_2^+ .X$, the orthoesters of formula $X1-(OR')_3$ and the nitriles of formula X1-CN, where X1 has the above mentioned meanings, X is halogen and R' is preferably an alkyl having low molecular weight. A preferred reactant is benzamidine.

The methods for preparing the Compounds of Formula I change according to the nature of the desired Compound. Excepting those where X1 and X2 together are an oxygen atom, they can be prepared according to known techniques.

Examples of known techniques for preparing oxazolines, i.e. the Compounds of Formula I where X2 and X3, together, are a covalent bond, are disclosed by E.H. Rodd ("Chemistry of Carbon Compounds" vol. IV; Heterocyclic Compounds, page 361, Elsevier Publishing Co. 1957) and by R.C. Elderfield ("Heterocyclic Compounds", vol. V, page 377, J. Wiley and Sons, Inc., 1957) as well as by Angew. Chem. Int. Ed. 15, 270-281, 1976; Chem. Rev. 71, 483506, 1971; Chem. Rev. 44, 447-476, 1941.

As mentioned before, the methods for preparing oxazolines involve the reaction of a compound of Formula

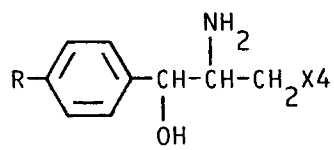

(III)

where R and X4 have the above mentioned meanings, with a suitable reactant such as an amidine, a halohydrate of an iminoether, an orthoester or a nitrile.

Alternatively they are prepared by dehydratation, catalyzed by acids, of amides of Formula

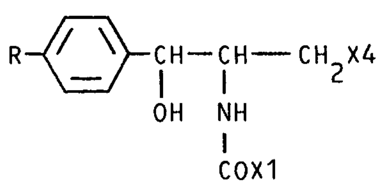

(IV)

or by cyclization, catalyzed by bases, of active esters of Formula

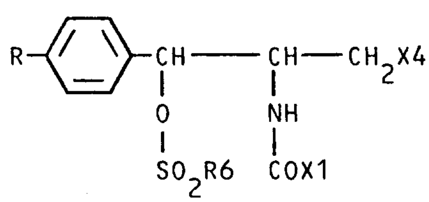

(IV)

where X1, X4 and R6 have the above mentioned meanings.

When X4 is -OH, the thus obtained oxazolines are then treated according to known techniques to afford the Compounds where X4 has the desired meaning.

The preparation of Compounds I where X1 and X2, together, are an oxygen atom is based on the unexpected finding that compounds of Formula

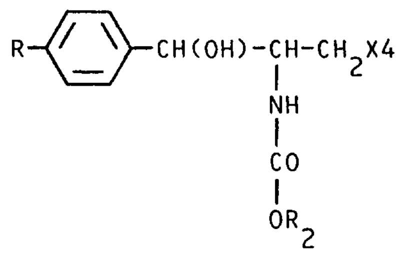

(V)

where R, X4 and R2 have the above mentioned meanings, cyclize regioselectively on the secondary hydroxy group to afford oxazolidininones of Formula I, in the presence of strong bases and of aprotic solvents.

The role of the solvent is critical. When the reaction is carried out in the presence of a non-aprotic solvent, the cyclization proceeds either on the secondary or on the primary hydroxy group and affords a mixture of the two possible cyclic compounds.

Examples of suitable aprotic solvents are the aromatic hydrocarbons such as benzene and toluene. Examples of suitable strong bases are the alkali and the alkaline earth metal alcoholates as well as the tertiary amines.

The cyclization reaction may also proceed via the intermediate formation of the alcoholate at the secondary hydroxy group when they are used alkali and alkaline earth metal alcoholates, alkali metal hydrides such as sodium hydride, sodium amide, Grignard-like organo-metallic derivatives and alkyl-lithium derivatives.

In their turn, the compounds (V) can be prepared according to known techniques such as the reaction of the desired 1-(phenyl)-2-amino-1,3-propanediol, substituted at the phenyl ring, with a compound of formula X-COOR2, where X is halogen and R2 has the above mentioned meanings, in the presence of a base and of a suitable diluent.

When it is used an organic diluent such as acetonitrile, it is preferably used an organic base such as a tertiary amine whereas is preferably used an inorganic base such as an alkali metal carbonate or bicarbonate, when the reaction is carried out in aqueous medium. Alternatively it may be used a basic diluent such as pyridine.

The compounds of formula V wherein R is $-SCH_3$ are more soluble into the aprotic solvents, than those wherein R is $-SO-CH_3$ or $SO_2CH_3$. A preferred way for preparing Compounds (I) wherein R is $-SO-CH_3$ or $-SO_2CH_3$ comprises the preparation of the corresponding Compounds (V) wherein R is $-S-CH_3$, their subsequent cyclization and lastly, their oxydation according to known techniques.

The substitution of the hydrogen of the primary hydroxy group with a OCOAlkyl(1-4C), trialkyl(1-6C)-silyl, tetrahydro pyranil, tetrahydrofuranyl and a $-SO_2R6$ radical, where R6 has the above mentioned meanings, can be carried out before or after the protection of both the secondary hydroxy and amino group. Also this substitution can be carried out according to known techniques.

The fluorination of Compounds I where X4 is not fluorine can be carried out either with DAST or with less expensive and more amenable fluorination agents such as FAR (1-diethylamino-1,1-difluoro-2-chloro-2-fluoro-ethane), phosphorus fluorides, hydrofluoric acid optionally salified with soluble or polymeric tertiary amines, alkali and alkaline-earth metal fluoride.

Examples of suitable fluorination agents when X4 is -OH are FAR, phosphorus fluorides and hydrofluoric acid.

When FAR is used, the reaction is carried out under anhydrous conditions and in homogeneous phase, preferably in acetonitrile at the boiling temperature.

Examples of desired fluorination agents when X4 is -O-COAlkyl(1-4C), -O-trialkylsilyl, -O-tetrahydropyranyl, -O-tetrahydrofuranyl or $-O-SO_2-R6$, where R6 has the above mentioned meanings, are the alkali and the alkaline-earth metal fluorides.

Anyway, the replacement of the group X4 with fluorine is a complex tecnical problem particularly when it is desired to use the most economic and amenable fluorination agents, that is the alkali and alkaline-earth metal fluorides, because F anion behaves besides as a nucleophilic agent also as a base causing

competitive side-reactions such as elimination, hydrolysis or, whenever it is possible, solvolysis (E.V. & S.M. Dehmlow "Phase transfer catalysis" Verlag Chemie, 1980, page 80).

Because of the peculiar nature of the hydrogen atom that is marked with an asterisk in Formula (I), the Compounds (I) undergo elimination. When they have been reacted with $F^{\ominus}$ in phase transfer conditions or in the presence of crown ethers, they did not react or did afford low yields of the desired compound owing to the concomitant formation of hydrolization and elimination products.

Now, it has been found that these drawbacks can be overcome when the reaction between a Compound (I) wherein X4 is -O-COAlkyl(1-4C), -O-trialkylsilyl, -O-tetrahydropyranyl, -O-tetrahydrofuranyl or -O-$SO_2$-R6, where R6 has the above mentioned meanings, and $F^{\ominus}$ is carried out in the presence of a suitable polyglycol. Preferably, the fluorination step will be carried out according to the last technique.

After performance of the fluorination step, the protective groups are removed from the Compounds of Formula I wherein X4 is fluorine.

A preferred method is consisting in removing the protective groups with acids, preferably inorganic acids, in aqueous medium or in water/organic diluents mixtures. The latter media are preferred when hydrolysis regenerates the compound or the compounds which had been previously used as protective agents and when the amine which is formed is soluble in an aqueous solution of inorganic acids. It is thus obtained a repartion of the amine in the aqueous layer and of the protective agent or agents in the organic layer, from which they are recovered and then recycled; in its turn, the amine is recovered by precipitation via neutralization of the aqueous layer. Alternatively the amine can be extracted with a suitable organic solvent.

When X1 and X2, together are an oxygen atom, the protective group may also be removed through treatment with an organo-metallic derivative such as a Grignard's derivative and the subsequent hydrolysis in mild conditions with inorganic acids in water or in water/organic solvent mixtures.

Another possible method for removing the protective group when X1 and X2, together, are an oxigen atom, comprises the reduction of the keto group and the subsequent hydrolysis in mild conditions as described above. This reduction is preferably carried out with complex hydrides such as sodium borohydride.

After removal of the protective groups it is obtained a compound of Formula

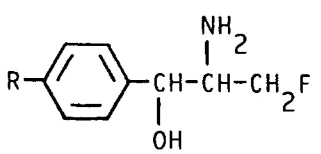

(VI)

which can be reacted with a haloacetic acid of Formula R1COOH, wherein R1 has the above mentioned meanings, or a reactive derivative thereof to afford the desired compound of Formula (II).

The protection performed by the Compounds of Formula I is useful not only for preparing the compounds of Formula II, but also whenever it is desired to replace the primary hydroxy group with another functional group, such as chlorine, bromine, iodine, nitrile, hydrogen, -$OR_7$, -$SR_7$, -$SCOR_7$, -SCN, -S(=NH)-$NH_2$, -CH-$(COOR_7)_2$, wherein R7 is alkyl, aralkyl or aryl.

This invention is illustrated by the following examples which should not be constructed as limiting it in any way.

EXAMPLE 1

Preparation of 3-acetoxy-1-(4-methylsulfonyl-phenyl)-2-phthal-imido-1-hydroxy-propane (A).

D-(threo)-1-(4-methylsulfonylphenyl)-2-phthalimido-1,3-propanediol (1 g; 2.66 mmols), prepared as described in U.S. Patent 4,235,892, has been dissolved in anhydrous pyridine (5 ml). Acetylchloride (0.2 ml; 2.83 mmols) has been added dropwise to this solution kept under stirring at O°C; after completion of the addition, the reaction mixture has been heated to 25°C and kept under stirring for 1 hour; afterwards, the reaction mixture has been poured into water and ice, acidified with hydrochloric acid and extracted with ethyl acetate.

The crude product (A) has been obtained (quantitative yield) from the organic layer after drying over sodium sulfate and evaporation of the solvent in vacuo; the crude, after crystallization from methanol, gave a pure product (0.84 g; yield 75%) as shown by HPLC and TLC analysis.

| Elemental Analysis for $C_{20}H_{19}O_9N$ | | | |
|---|---|---|---|
| (found)<br>(Calculated) | C 57.3 %;<br>C 57.55%; | H 4.6 %;<br>H 4.56%; | N 3.3 %<br>N 3.36%. |

The acetylation is regio-selective on the secondary hydroxy group as shown by NMR spectrum in DMSO; delta = 1.78; s, 3H, $CH_3CO$-; 4.50 dd- 2H, -$CH_2OAc$; 6.02,d,1H, benzylic OH.

EXAMPLE 2

Reduction of compound (A) to 3-acetoxy-1-(4-methylsulfonylphenyl)-1-hydroxy-2-(3-hydroxy-1H-isoindol-1-one-2-yl)-propane (B).

Compound (A) (0.76 g; 1.82 mmols) has been added to a mixture of tetrahydrofuran and water (1:1; 4 ml); to this suspension, kept at 0°C under vigorous stirring, has been added portionwise sodium borohydride (138 mg; 3.64 mmols).

As the reaction proceeded, the suspension became a homogeneous solution, after 1 hour and after having checked by TLC the disappearance of compound (A) and the formation of a new product, tetrahydrofuran has been evaporated in vacuo and the product extracted with ethyl acetate.

After drying over sodium sulfate and evaporation of the solvent, compound (B) (0.7 g; yield 92%) has been obtained sufficiently pure to undergo as such the following reaction (Example 3).

Compound (B) proved to be a mixture of two diastereoisomers because of the formation, during the reduction step, of a new asymmetric carbon atom; this has been proved by TLC, HPLC and NMR spectra in DMSO containing $D_2O$; delta 1.78; s, and 1.86, s, 3H on the whole, $CH_3$-CO in two diastereoisomers in the ratio 35:65; 5.84,s, and 6.24,s,1H, on the whole

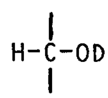

of the isoindole system (doublets, before deuteration, coupled with two doublets ehibiting delta = 6.8 and 6.57 respectively, 1H on the whole for -OH in the two diastereoisomers) and, finally, 5.14, d, and 5.2, d, 1H on the whole for the two

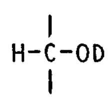

in position 1 of the propane chain.

EXAMPLE 3

Cyclization of compound (B) to 3-acetoxymethyl-2-(4-methylsulfonyl-phenyl)-2,3-dihydro-oxazole-[2,3,a]-isoindol-5(9bH)-one (C).

Product (B) (0.55 g; 1.3 mmol) has been suspended in benzene (5 ml) containing a little amount of p-toluensulfonic acid (5 mg); by heating, the mixture became a clear solution. A short time later the water formed during the reaction has been distilled azeotropically until water was absent in the distilled benzene and TLC analysis showed the disappearance of product (B). At the end, almost all benzene has been evaporated in vacuo; after having added some water, product (C) has been extracted with ethyl acetate. Crude product (C) has been obtained in quantitative yield from the organic phase after drying over sodium sulfate and evaporation of the solvent in vacuo.

Crude product (C) has been used as such for the subsequent hydrolysis (Example 4). An aliquot has been purified by chromatography on silica gel using ethyl acetate/petroleum ether in various ratios or pure ethyl acetate as eluents. It has been proved that the crude contained small amounts of some unidentified impurities; after purification by chromatography has been proved to be a mixture of two diastereoisomers as shown by TLC and HPLC analysis as well as by NMR spectrum in DMSO: delta 6.38,s, and 6.07 s, 1H on the whole,

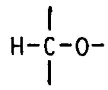

of the isoindole system; 2.08 s and 2.18 s, 3H on the whole, $CH_3CO$-; 3.19, s and 3.24, s, 3H on the whole, $CH_3SO_2$-.

| Elemental analysis for $C_{20}H_{19}O_8N$ | | | |
|---|---|---|---|
| (calculated):<br>(found) | C, 59.85%;<br>C, 59,9 %; | H, 4.74%;<br>H, 4.6 %; | N, 3.49%<br>N, 3.6 % |

EXAMPLE 4

Hydrolysis of compound (C) to 2-(4-methylsulfonyl-phenyl)-3-hydroxy-methyl-2,3-dihydro-oxazole-[2,3,a]-isoindol-5(9bH)-one (D).

Product (C) (0.2 g; 0.5 mmols) has been dissolved in methanol (2 ml) containing potassium hydroxide (42 mg; 0.75 mmols) at 0 ° C and under vigorous stirring. After 30' the hydrolysis has been checked by TLC and showed the disappearance of product (C).

Methanol has been evaporated in vacuo and in the cold; the residue has been treated with water and extracted with ethyl acetate. The organic layer has been dried and evaporated to afford product (D) which has been recrystallized from ethyl acetate (0.16 g; yield 89%). The presence of two diastereoisomers in product (D) has been shown by NMR spectrum in DMSO, delta = 6.3, s and 5.82,s, 1H on the whole,

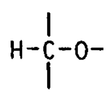

of the isoindole system; 3.18,s, and 3.22,s, 3H on the whole, $CH_3SO_2$-.

EXAMPLE 5

Preparation of 2-(4-methylsulfonyl-phenyl)-3-fluoromethyl-2,3-dihydroxazole-[2,3,a]-isoindol-(9bH)-one (E).

35 ml of anhydrous acetonitrile, cooled to 0 ° C, has been added with 3.2 ml (20 mmols) of FAR (1-diethylamino-1-difluoro-2-chloro-2-fluoroethane). After 10 minutes compound carefully dried (D) (5 g; 13.9 mmols) has been added portionwise; when the addition has been over, the solution has been refluxed for 2 hours. After completion of the reaction, the solvent has been evaporated in vacuo and the residue, after treatment with water and ice, has been extracted with ethyl acetate. The organic layer has been dried over sodium sulfate and evaporated in vacuo to afford crude (E), which has been used as such for the subsequent hydrolysis (Example 6) an aliquot has been purified by chromatography on silica gel and the diastereoisomer present in the mixture in biggest amount has been isolated and showed a very high purity degree. NMR spectrum in $CHCl_3$:delta = 3.04,s,3H, $CH_3SO_2$; 4.18,m,1H,

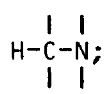

5.58, d,, 1H, J = 6 cps,

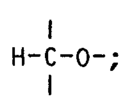

6.20, s, 1H,

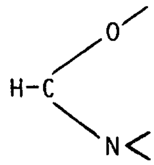 ;

4.82, m, 2H,

$J_{H_1F}$ 48Hz =

EXAMPLE 6

Hydrolysis of compound (E) and preparation of D-threo-1-(4-methylsulfonyl-phenyl)-1-hydroxy-2-dichloroacetamido-3-fluoropropane (F).

Compound (E) (2.07 g; 5.73 mmols) has been suspended in 2N HCl (60 ml) and the suspension refluxed for 7 hours.

After cooling, the mixture has been extracted with ethyl ether to recover the phthalaldehydic acid formed during the hydrolysis step.

The aqueous layer has been saturated with sodium chloride and potassium carbonate, extracted with ethyl acetate and then with chloroform.

The combined organic extracts have been dried over sodium sulfate and evaporated in vacuo. The crude product (F) has been thus obtained, which, without further purification, has been treated, at the boiling temperature for 6 hours, with methyl dichloroacetate (6 m) in the presence of catalytic amounts of triethylamine. After completion of the reaction, the volatile compounds have been removed and the residue has been chromatographied on silica gel while collecting the fractions containing (F). Compound (F) has been compared with a sample obtained according to another process (U.S. Patent 4,235,892) and proved, by analytical and microbiological tests, to be identical with the sample.

EXAMPLE 7

Preparation of 2-(4-methylsulfonyl-phenyl)-3-methan sulfonyloxymethyl-2,3-dihydro-oxazole[2,3,a]-isoindol-5-(9bH)-o ne (G).

Freshly distilled methanesulfonyl chloride (0.35 ml; 4.59 mmols) has been added to a solution of compound (D) (1.5 g; 4.17 mmols) in pyridine (3 ml), kept at 0 ° C and under stirring. The mixture was allowed to stand in refrigerator overnight and then added with ice and extracted with ethyl acetate. The combined organic extracts have been dried over sodium sulfate and the solvent removed by evaporation.

Compound (G) has been thus obtained in a purity degree sufficient to undergo the subsequent reaction (Example 8).

EXAMPLE 8

Preparation of compound (E) from (G).

Compound (G) (1.48 g; 3.38 mmols) has been dissolved in warm toluene (7 ml). This solution was added with an aqueous solution of potassium fluoride (4 ml; solution consisting of 3 g of potassium fluoride and 3 g of water) and with hexadecyl tributylphosphonium chloride (0.28 g).

The thus obtained heterogeneous mixture has been refluxed under vigorous stirring for 7 hours. The organic layer has been then separated and evaporated; the residue has been treated with water and extracted with ethyl acetate. The organic layer has been dried over sodium sulfate and evaporated to afford crude product (E) which has been purified by chromatography on silica gel. The thus obtained product showed the same characteristics as the product obtained according to Example 5.

EXAMPLE 9

Preparation of 1,3-dihydroxy-1-(4-methylthio-phenyl)-2-ethoxycarbonylamino-propane (H).

D-(threo)-1-(4-methylthio)-phenyl-2-amino-1,3-propanediol (1.06 g; 4.93 mmols) has been suspended into an aqueous solution of potassium carbonate (1.8 f of potassium carbonate into 20 ml of water) and the thus obtained mixture has been cooled, under vigorous stirring, to 0 ° C.

Ethyl chlorocarbonate (0.5 ml) has been dropped quickly into the mixture maintained under vigorous stirring at 0 ° C; after half a hour, further 0.24 ml of ethyl chlorocarbonate (total amount: 7.74 mmols) has been added and the mixture has been maintained under stirring for 1 further hour.

At first the reaction mixture became clear and then a white precipitate has been slowly formed. After having checked the completion of the reaction by TLC, the suspension has been extracted with ethyl acetate. After drying on sodium sulfate, filtration and evaporation of the solvent, the organic extracts afforded 1.37 g of crude (H) (yield, 95.5%) which has been recrystallized from ethyl acetate/diisopropyl ether. m.p. = 75 ° C.

I.R. spectrum: 3340 and 3450 $cm^{-1}$ (OH, NH stretching),

1690-1700 $cm^{-1}$ (broad band: C = O amide).

In a similar manner it has been prepared the 1,3-dihydroxy-1-(4-methylsulfonyl-phenyl)-2-ethoxycarbonylamino-propane (I) which, after crystallization from ethyl acetate, showed (IR analysis) the following peaks: 3200-3360 cm (broad band - OH and NH stretching), 1715 cm (CO amide).

EXAMPLE 10

Cyclization of compound (H) to 5-(4-methylthio-phenyl)-4-hydroxy-oxazolidin-2-one (J).

Compound (H) (5 g; 17.5 mmols) has been dissolved in warm toluene (25 ml). To this solution, an equimolar amount of potassium ter.butylate has been added and the reaction mixture has been refluxed for 3 hours. Afterwards, almost all the solvent has been evaporated; water and ice have been added to the residue and the precipitate has been collected by filtration. The thus obtained crude (J) has been recrystallized from ethanol (3.7 g; yield, 88%); m.p. 130-131 ° C.

I.R. Spectrum: 3180, 3240, 3300 $cm^{-1}$ (OH and NH stretching)

1720; 1745 $cm^{-1}$ (C = O, oxazolidinone);

NMR in DMSO, delta: 7.64 and 8.0, two doublets, 2H each one of p-substituted phenyl; 7.88, S, 1H, NH amido; 5.48, d, 1H, benzyl hydrogen; 3.56, m, 2H, hydroxymethyl; 5.16, m, H linked to C4 of oxazolidinone ring; 3.2, S, 3H, $CH_3S$-.

EXAMPLE 11

Oxidation of compound (J) to 5-(4-methylsulfonyl-phenyl)-4-hydroxymethyl-oxazolidin-2-one (K).

Compound (J) (53 g; 221 mmols) has been added portionwise to 84 ml of hydrogen peroxide (130 vol.) maintained under stirring at 40-45 ° C. After completion of the addition, the stirring has been continued for further 20 hours at 40 ° C.

Acetic anhydride (76.6 g; 20.7 ml) has been dropped into the reaction mixture by keeping the temperature below 40 ° C.

The reaction mixture has been then cooled to 20-22 ° C and maintained under stirring at this temperature for 3 hours and, finally, allowed to stand in refrigerator overnight.

Afterwards, the solvent has been evaporated with caution in vacuo at 40 ° C; hot ethanol has been added to the thus obtained residue. The solvent has been again evaporated and the residue crystallized from methyl alcohol. 48.6 g of product (K) yield, 81%. m.p. 172-174 ° C.

I.R. spectrum: 1710 $cm^{-1}$ (C = O, amide)

3470, 3340, 3250, 3200 $cm^{-1}$ (OH and NH).

EXAMPLE 12

Preparation of 5-(4-methylsulfonyl-phenyl)-4-fluoromethyloxazolidin-2-one (L) from (K).

To 1 ml of acetonitrile at 0 ° C have been added two drops of FAR and, after some time, anhydrous compound (K) (100 mg; 0.42 mmols); afterwards, has been added the remaining aliquot of FAR (total amount: 1.5 mols to each mole of compound K); the suspension has been maintained under stirring at 0 ° C for 10 minutes and the temperature has been then allowed to rise up to 20 ° C. When the suspension became clear, has been refluxed for 3 hours. After evaporation of the solvent, the residue has been treated

with water and ethyl acetate. The organic extract has been dried over sodium sulfate, evaporated to dryness and the residue (90 mg) chromatographied on silica gel. Pure product (L) has been thus obtained (45 mg).
I.R. spectrum: 1750 $cm^{-1}$ (C = O, oxazolidinone)
NMR in DMSO, delta: 7.70 and 8.05, two doublets, 2H each one of 4-substituted phenyl; 8.16, S, NH amido; 5.6, d, 1H, benzyl hydrogen; 4.74 and 4.5, two multiplets, 1H each.

EXAMPLE 13

Preparation of 5-(4-methylsulfonyl-phenyl)-4-methanesulfonyloxy-methyl-oxazolidin-2-one (M) from (K).

Compound (K) (200 mg; 0.84 mmols) has been dissolved in anhydrous pyridine (3 ml). The thus obtained solution has been cooled to 0 ° C and added with freshly distilled methanesulfonyl chloride (0.06 ml). The solution has been maintained at 0 ° C overnight and then diluted with an aqueous solution of hydrochloric acid (stoichiometric amount with respect to pyridine) and ice. After extraction with ethyl acetate, the organic extract has been dried over sodium sulfate and evaporated in vacuo to afford crude (M); yield, 85-90%.
I.R. spectrum: 3300 $cm^{-1}$ (NH), 1760 and 1740 $cm^{-1}$.

EXAMPLE 14

Preparation of (L) from (M).

Product (M) (155 mg; 0.44 mmols) has been suspended into 0.5 ml of toluene; to this solution have been added hexadecyltributylphosphonium bromide (22.3 mg) and a concentrate solution of KF (207 mg; 2.2 mmols) in water.

The reaction mixture has been refluxed under vigorous stirring for 6 hours and then diluted with water. The aqueous layer has been extracted with ethyl acetate; the combined organic extracts have been dried over sodium sulfate, evaporated in vacuo and the crude residue (110 mg) has been chromatographied on silica gel to afford 40 mg of pure product (L).

EXAMPLE 15

Preparation of D(-)-threo-2-phenyl-4-hydroxymethyl-5-(4-methylthiophenyl)-2-oxazoline (N).

i) D-(-)-threo-1-(4-methylthiophenyl)-2-amino-1,3-propane diol (20 g; 94 mmols) has been thoroughly mixed with benzamidine hydrochloride dihydrate (25 g; 130 mmols) and melted at 100 ° C. The melted mass, after 1 hour at said temperature has been cooled.

To the cooled mass has been added ethyl alcohol (850 ml) and the mixture has been refluxed till complete dissolution. The solution has been then filtered with active carbon and cooled to -10 ° C. The crystalline precipitate has been collected by filtration and dried at 50 ° C under reduced pressure for 10 hours.

19 g (69%) of compound (N) have been thus obtained, m.p. 174-176 ° C.

The thus obtained product has been purified by crystallization from ethyl alcohol (400 ml), filtration and drying at 60 ° C under reduced pressure for 8 hours.

Yield, 17 g (62%); m.p. 175-177 ° C.

ii) A solution of 390.6 g (1.83 moles) of D-(-)-threo-1-(4-methylthiophenyl)-2-amino-1,3-propanediol and 374 g (2.01 moles) of ethyl benzimidate hydrochloride in 500 ml of water was warmed under stirring at 30 ° C for 20 hours.

The mixture was cooled at 5 ° C and the resulting precipitate was filtered, washed with water and dried in vacuum at 50 ° C.

The crude oxazoline was purified by recrystallization from ethanol to give 395 g (72%) of product (N), m.p. 172-73 ° C.

In a similar manner may be prepared from D-(-)-threo-1-(4-methylthiophenyl)-2-amino-1,3-propanediol and the proper imidate the following derivatives:
D-(-)-threo-2-(4-nitrophenyl)-4-hydroxymethyl-5-(4-methylthio phenyl)-2-oxazoline (O)
D-(-)-threo-2-benzyl-4-hydroxymethyl-5-(4-methylthiophenyl)-2-oxazoline (P).

EXAMPLE 16

Preparation of D-(-)threo-2-methyl-4-hydroxymethyl-5-(4-methylthiophenyl)-2-oxazoline (Q).

Ethyl acetoimidate hydrochloride (50 g; 400 mmols) has been suspended in dichloromethane (360 ml). To the thus obtained suspension has been added D-(-)-threo-1-(4-methylthiophenyl)-2-amino-1,3-propanediol (76.37 g; 358 mmols). The reaction mixture has been stirred for 5 hours and then poured into ice and water (230 g).

The aqueous layer has been separated and extracted with dichloromethane (60 ml). The combined organic extracts have been washed with water, dried on sodium sulfate and evaporated to dryness.

Crystalline product (Q) (69.50 g; 81%) has been thus obtained which has been purified by crystallization from methyl-ter.butyl-ether (1750 ml), filtration and drying in vacuo at 40-50 °C.

Yield, 55.60 g (65%) m.p. 111-113 °C.

In a similar manner has been prepared D-(-)-threo-2-methyl-4-hydroxymethyl-5-(4-methylsulfonylphenyl)-2-oxazoline (R) melting at 153-155 °C.

EXAMPLE 17

Preparation of D-(-)-threo-2-methyl-4-acetoxymethyl-5-(4-methyl thiophenyl)-2-oxazoline (S).

A mixture of D-(-)-threo-1-(4-methylthiophenyl)-2-acetyl amino-3-acetoxy-propanol (10 g; 33.6 mmols), 4-toluensulfonic acid (0.5 g) and toluene (100 ml) has been refluxed for two hours and a half in a round-bottomed flask equipped with a water trap.

After cooling, the reaction mixture has been added with water (50 ml). The organic layer has been separated, washed with water till neutral, dried over sodium sulfate and evaporated to dryness.

The residue has been treated with hot hexane (3 x 80 ml) while decanting the solvent each time. The combined organic extracts have been concentrated to 90 ml, added with celite (5 g) and filtered through a hot glass funnel. By cooling has been obtained a crystalline precipitate (S) which has been collected by filtration and dried in vacuo.

Yield, 3.60 g (38.5%); m.p. 62-64 °C.

In a similar manner may be prepared the following derivatives:

D-(-)-threo-2-ethyl-4-acetyloxymethyl-5-(4-methylsulfonylphenyl)-2-oxazoline (T).

EXAMPLE 18

Preparation of D-(-)-threo-2-phenyl-4-hydroxymethyl-5-(4 methylsulfonylphenyl)-2-oxazoline (U).

D-(-)-threo-2-phenyl-4-hydroxymethyl-5-(4-methylthiophenyl)-2-oxazoline (5 g; 16.7 mmols) prepared as disclosed by Example 15 above, has been added portionwise to acetic anhydride (5.80 ml) under stirring at 35 °C. Into the thus obtained suspension, has been dropped hydrogen peroxide (20 ml; 130 volumes) maintaining the temperature at 35-40 °C by external cooling.

After completion of the addition, stirring has been continued at 35 °C till complete dissolution and the solution is allowed to stand overnight at 4 °C. To the reaction mixture has been added water (25 ml). The precipitate has been separated by filtration, washed on the filter till neutral and then dried in vacuo at 60 °C to afford 4.4 g of the desired product (U) which has been purified by crystallization from methyl alcohol (400 ml).

Yield, 3.80 g (68.5%); m.p. 209-211 °C.

EXAMPLE 19

Preparation of D-(-)threo-2-dichloromethyl-4-methanesulfonyloxymethyl-5-(4-methylsulfonylphenyl)-2-oxazoline (V).

Methanesulfonyl chloride (8.13 ml; 105 mmols) has been dropped in 15 minutes into a solution of D-(-)-threo-1-(4-methylsulfonylphenyl)-2-dichloroacetylamino-1,3-propane diol (35.60 g; 100 mmols) in anhydrous pyridine (136 ml) maintained under stirring at 0 °C.

The reaction mixture has been kept under stirring for 1 further hour and then allowed to stand in refrigerator overnight.

The thus obtained mixture has been poured into a mixture of concentrate hydrochloric acid (150 ml) and ice and extracted with ethyl acetate (500 ml x 2).

The combined organic extracts have been washed with 5% hydrochloric acid (200 ml) and then with water till neutral, dried over sodium sulfate and evaporated to dryness.

The residue has been purified by crystallization from ethyl alcohol (200 ml).

24.4 g (56%) of D-(-)-threo-1-(4-methylsulfonylphenyl)-2 dichloroacetylamino-3-methanesulfonyloxypropanol melting at 109-112 °C have been thus obtained.

A mixture of this product (2 g; 4.6 mmols) and of methane sulfonic acid (0.1 ml) in 35 ml of 1,2-dichloroethane, has been refluxed in a round-bottomed flask equipped with a water trap for 3 hrs. The reaction mixture has been cooled and washed with water.

The organic layer has been separated, dried over sodium sulfate and evaporated to dryness. The residue (0.7 g) has been purified by chromatography on a silica gel column (70 g) eluting with dichloromethane/methyl alcohol (9.5:0.5). The fraction containing the desired product (V) has been isolated and evaporated to dryness.

It has been thus obtained a product (200 mg) melting at 116-118 °C which show a NMR spectrum consistent with the structural formula.

In a similar manner may be prepared from D-threo-(1-(4-methylsulfonylphenyl)-2-trifluoroacetylamino-1,3-propanediol (British Patent No. 1534387) the correspondent D-(threo-(-)-2-trifluoromethyl-4-hydroxymethyl-5-(4-methylsulfonylphenyl)-2-oxazoline (W).

EXAMPLE 20

Preparation of D-(-)-threo-2-phenyl-4-methanesulfonyloxy methyl-5-(4-methylthiophenyl)-2-oxazoline (X).

A mixture of product (N) (4 g; 13.37 mmols) prepared as disclosed in Example 15 above, in anhydrous pyridine (15 ml) has been kept under stirring at room temperature for about 1 hr. After cooling to 0 °C has been added dropwise methane sulfonyl chloride (1145 ml; 14.7 mmols) and stirring has been continued for 2 further hrs maintaining the temperature at 0 °C. The mixture has been then allowed to stand at about 3 °C for 3 hrs. Ice has been added to the reaction mixture and the thus obtained solid has been filtered and dried in vacuo at room temperature.

4.96 g of chromatographically (T.L.C.) pure product (T) have been thus obtained.

Yield, 98%; m.p. 107-108 °C.

In a similar manner may be prepared the following compounds:

D-(-)-threo-2-methyl-4-methanesulfonyloxymethyl-5-(4-methylthiophenyl)-2-oxazoline (Y)
D-(-)-threo-2-benzyl-4-methanesulfonyloxymethyl-5-(4-methylthiophenyl)-2-oxazoline (Z)
D-(-)-threo-2-trifluoromethyl-4-methanesulfonyloxymethyl-5-(4-methylsulfonylphenyl)-2-oxazoline (a)
D-(-)-threo-2-(4-nitrophenyl)-4-methanesulfonyloxymethyl-5-(4-methylthiophenyl)-2-oxazoline (b).

EXAMPLE 21

Preparation of D-(-)-threo-2-phenyl-4-fluoromethyl-5-(4-methylthiophenyl)-2-oxazoline (c).

Product (X) (4 g; 10.61 mmols) prepared as disclosed in Example 20 and anhydrous potassium fluoride (6.164 g; 106 mmols) in polyethylene glycol 400 (25 ml) have been maintained under stirring at 100-105 °C for 19 hrs. After having added water, the mixture has been extracted with ethyl ether (3 x 10 ml). The combined organic extracts have been dried over sodium sulfate and evaporated to dryness. The oily residue has been purified by flash chromatography on a silica gel column eluting at first with 900 ml of ethyl acetate/petroleum ether 1:9 and then with 1500 ml of ethyl acetate/petroleum ether 2:8.

2.19 (yield, 68.5%) of product (c) have been thus obtained.

An analitically pure sample has been prepared by crystallization from isopropanol; m.p. 70-72 °C.

NMR ($CDCl_3$) delta: 5.5 d, (J = 7Hz) (H-C5); 5.08, d.d, (1H)
($CH_2F$); 4.13-4.71, m, 2H (H-C4 and $-CH_2F$).

EXAMPLE 22

Preparation of D-(-)-threo-1-(4-methylthiophenyl)-2-amino-3-fluoro-1-propanol (d).

Product (c) (1.01 g; 3.35 mmols) prepared as disclosed in Example 21 has been added to 2N hydrochloric acid (18 ml) and maintained under stirring at 100-105 °C. After 50 minutes, further 20 ml of 2N hydrochloric acid have been added and the stirring is continued at 100-105 °C. After 3 hrs and a half the solution has been cooled and extracted with ethyl ether (20 ml x 2). The aqueous layer has been at first made basic with sodium bicarbonate and the satured with potassium carbonate. Finally it has been extracted with chloroform (20 ml x 3). The combined organic extracts have been evaporated to dryness in vacuo.

0.64 g of product (d) have been thus obtained. Yield, 88%.

EXAMPLE 23

Preparation of D-(-)-threo-2-phenyl-4-methanesulfonyloxy methyl-5-(4-methylsulfonylphenyl)-2-oxazoline (e).

To a mixture of product (U) (0.3 g; 0.9 mmols) prepared as disclosed in Example 18, in pyridine (5 ml) has been added methanesulfonyl chloride (0.077 ml; 0.99 mmols) at 0 °C.

After having continued the stirring at 0 °C for 10 minutes, the mixture has been allowed to stand in refrigerator overnight. The mixture has been then made acid, extracted with ethyl acetate (15 ml x 2), dried over sodium sulfate and evaporated to dryness in vacuo.

0.32 g of product (e) have been thus obtained. Yield, 87%.

EXAMPLE 24

Preparation of D-(-)-threo-2-phenyl-4-fluoromethyl-5-(4-methylsulfonylphenyl)-2-oxazoline (f).

Product (e) (0.32 g; 0.78 mmols) prepared as disclosed in Example 23 above and anhydrous potassium fluoride (0.53 g; 0.91 mmols) in polyethylene glycol (400 ml) have been kept under stirring at 90-95 °C for 3 hrs, then at 75 °C for 17 hrs and, finally, at 110 °C for 6 hrs. The reaction mixture has been worked up as disclosed in Example 21 and have been thus obtained 0.13 g of product (f). Yield, 50%.
NMR ($CDCl_3$) delta: 5.76 d, (J = 7Hz, H-C; 5.16, d.d, (1H) ($-CH_2F$); 4.2-4.75, m, (2H), (H-C4 and $-CH_2F$).

## Claims

**1.** A process for preparing a D-threo compound of the formula

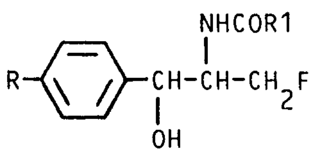

(II)

wherein

R is $CH_3S$-, $CH_3SO$-, or $CH_3SO_2$; and
R1 is mono-, di-, and tri-halomethyl,

characterized in that

(i) both the secondary hydroxy and the amino group of a corresponding D-threo 1-(phenyl)-2-amino-1,3-propanediol are protected via a D-threo compound of the formula

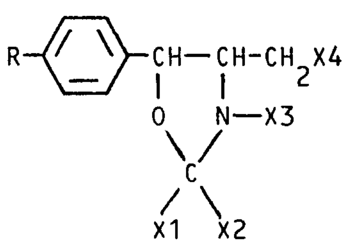

(I)

where

R has the above mentioned meanings;
X1 is 1-6 C haloalkyl;
X2 together with X3 is a covalent bond;
X4 is -OH, OCOAlkyl(1-4C), -O-trialkyl(1-6C) silyl, -O-tetrahydropyranyl, -O-tetrahydrofuranyl or $-O-SO_2R6$ where R6 is methyl, trifluoromethyl, phenyl or p-methyl-phenyl;

(ii) the D-threo compound of formula (I) is fluorinated to afford a D-threo compound of the formula

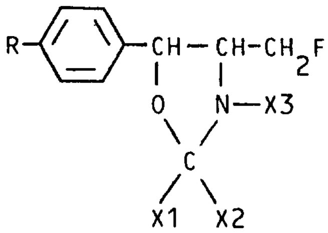

(Ia)

where
R, X1, X2 and X3 have the above mentioned meanings;
(iii) the D-threo compound of formula (Ia) is treated to afford a D-threo compound of the formula (II).